Europäisches Patentamt

European Patent Office

Office européen des brevets

(19)

(11) Publication number: **0 084 435**
**B1**

## EUROPEAN PATENT SPECIFICATION

(12)

(45) Date of publication of patent specification: **27.08.86**

(21) Application number: **83300159.7**

(22) Date of filing: **13.01.83**

(51) Int. Cl.⁴: **G 02 B 23/08, A 61 B 1/04,**
**A 61 B 1/00, G 02 B 23/06**

(54) **Endoscopes having a focus state detector.**

(30) Priority: **14.01.82 JP 4115/82**
**14.01.82 JP 4116/82**
**02.06.82 JP 94275/82**

(43) Date of publication of application:
**27.07.83 Bulletin 83/30**

(45) Publication of the grant of the patent:
**27.08.86 Bulletin 86/35**

(84) Designated Contracting States:
**AT BE CH DE FR GB IT LI NL SE**

(56) References cited:
**EP-A-0 080 340**
**DE-A-3 021 887**
**DE-A-3 148 325**
**US-A-4 153 834**

(73) Proprietor: **OLYMPUS OPTICAL CO., LTD.**
**43-2, 2-chome, Hatagaya Shibuya-ku**
**Tokyo 151 (JP)**

(72) Inventor: **Nakamura, Takeaki c/o Olympus**
**Optical Co. Ltd.**
**No. 43-2, Hatagaya 2-chome**
**Shibuya-ku Tokyo (JP)**
Inventor: **Shishido, Yoshio c/o Olympus Optical**
**Co. Ltd.**
**No. 43-2, Hatagaya 2-chome**
**Shibuya-ku Tokyo (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

Courier Press, Leamington Spa, England.

# Description

This invention relates to endoscopes having a focus state detector, means being provided for projecting a light beam of high intensity through a spot illuminating light guide.

Recently endoscopes have become extensively used in both the therapeutic field and the industrial field. Particularly in the therapeutic field, it is a common practice for diagnostic purposes to directly observe an organ or the like within a body cavity by the eye, through an eyepiece of an endoscope, and to record an optical image for diagnosis by fitting a photographic or television camera.

In the case of observation with the naked eye, a clear optical image can be formed, that is, as the state of focus will be apparent to the technician when he is adjusting the instrument, but if a photographic or television camera is fitted, to obtain a clear optical image it is necessary to ensure that the lens is correctly adjusted to a correct focus setting.

The light reflected to form an optical image from an object such as an organ within a body cavity is generally so low in intensity and the contrast so poor that, in most cases of recording an image of an object only a short distance from the entry plane of the optical system, the depth of focus will become so shallow that it is very difficult to obtain a correct focus state. Therefore, the recorded image is often found to be out of focus, and means by which the correctly focused state can be positively detected in an endoscope has been long desired.

In our earlier Specification published in the United States of America as Serial No 4 153 834 on May 8th 1979, an automatic focusing system is described for an endoscope using an optical fibre viewing system for projecting a focusing spot when the illuminating source is reduced or put out in one embodiment, or used simultaneously, but obliquely in a second embodiment.

Our earlier Specification published in German 30 21 887 also shows separate light sources for focusing and normal illumination.

Our earlier Application published as EP—A—0 080 340 particularly relates to the transducer design in such systems, and no lens is provided between the optical fibre branch and the mirror-surface on a common optical axis in embodiments employing fibre-optic branching to provide focusing-spot illumination.

One object of the present invention is to provide an endoscope having a focus state detector, using means projecting a spot of illuminating light of high intensity to ensure that even if a dark optical system is used a focusing spot will be clearly detectable with high precision, using a simple structure of low cost contained in a small space.

According to the present invention there is provided an endoscope having a focus state detector, means being provided to transmit illuminating light from a light source to a light-beam transmitting means within an endoscope via a light guide and a light-spot guide to project a light-spot on to an object that is to be recorded as an optical image, the image of the illuminated object being passed back to a predetermined image forming surface via an observing optical system in the endoscope and thence through an adjustable lens system whose focusing state is monitored by said detector, which comprises a light receiving means using a photo-electric transducer element to assess whether there is a clearly focused image at said image forming surface, the light returning from said projected light-spot being directed to an optical aperture or locally non-sensitive part of said transducer having a shape and size matching that of the reflected light-spot formed in a position optically conjugate with said image forming surface, the light-emitting end face of said light-spot guide either facing an optical aperture formed in the light receiving means or an optical aperture adjacent to the light receiving means, characterised in that the light-spot guide is formed by a branching of the light guide that projects its light via a lens common to the path of the returning light.

The invention will now be described with reference to the drawings, in which:—

Figure 1 is an explanatory view showing an optical system of a focus state detector within an endoscope in a first exemplary embodiment, where the detector is contained in a camera adaptor;

Figure 2 is a plan view showing the shape of a photoelectric element used in the embodiment shown in Figure 1;

Figure 3 is a schematic explanatory view showing the focus detector mode of operation, with the optical system of the embodiment shown in Figure 1 converted to a linear optical system for the sake of simplicity;

Figure 4 is a graph showing the changes in output characteristics of a photoelectric element as the position of an object to be photographed via the optical system shown in Figure 3 is varied;

Figure 5 is a block schematic diagram showing the circuit arrangement provided in the embodiment shown in Figure 1 for focus state detection, and for setting an objective lens in a correctly focused position if an automatic control of focus and exposure is provided;

Figure 6 is an explanatory view showing an optical system of a second exemplary embodiment of an endoscope with a focus state detector;

Figure 7 is a schematic explanatory view showing the focus state detecting principle with the optical system of the embodiment shown in Figure 6 converted to a linear optical system for the sake of simplicity; and

Figure 8 is an explanatory graph showing the variation of output characteristics of a photoelectric transducer element as the position of the object to be recorded is varied in the optical system shown in Figure 7.

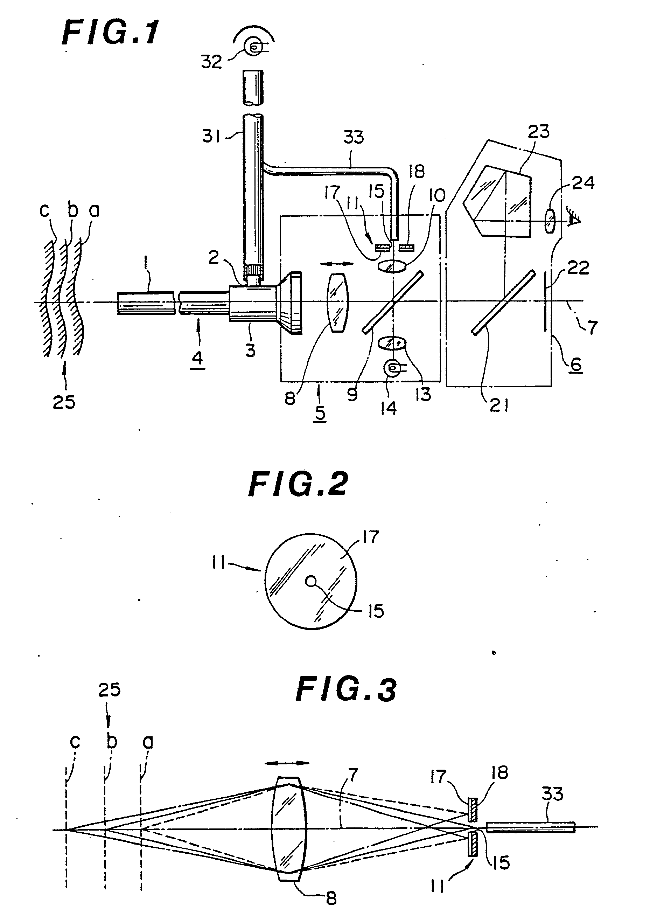

In the embodiment shown in Figure 1, a camera adaptor 5 for a photographic camera is removably

fitted to the rear end side of an eyepiece 3 of an endoscope 4 formed with an elongate insertion sheath 1 housing an objective optical system and illuminating optical system extending to the front tip, and leading to the eyepiece 3 at the outer end of the insertion sheath 1, where a light-guide mouthpiece 2 is provided on the side. A photographic or television camera 6 is attached via an adaptor 5 to be removably fitted to the rear.

An endoscope focus setting device whereby the objective lens of the optical system can be automatically moved and set at a correctly focused position by using the output of the endoscope focus state detector of the present invention is contained in the camera adaptor 5.

An objective lens 8, movable forward and rearward on an optical axis 7, as indicated by arrows, is arranged facing the eyepiece lens system of the endoscope 4 on the optical axis when the endoscope 4 is fitted in place. A semi-transparent focusing mirror 9 inclined (for example, by 45 degrees) to the optical axis 7, is arranged on the optical axis 7 to the rear of the lens 8.

An image reforming lens 10 and a photo-electric transducer element 11 are arranged as a light receiving means on a reflected optical axis (to the upper position as drawn in Figure 1) on which the returning light incident along the above mentioned optical axis 7 is reflected by the focusing mirror 9, so that the projected light and returning light both pass through this common lens 10, as can be seen from the drawings. A lamp lens 13 and focus indicating lamp 14 indicating focusing are arranged in turn on the reflected optical axis (in the lower position as drawn in Figure 1) to the rear side of the focusing mirror 9.

The photo-electric transducer element 11 is in the form of a plate, which may be a disc, or a square or rectangular element, and has provided an aperture 15 of a small shape such as a pinhole or a rectangular slit at its centre, as shown in Figure 2. On the front face of this disc plate there is formed a photo-electric surface 17 of a photo-sensitive material, for example, a photo-diode, photo-transistor or a photo-cell (e.g. of CdS), or an element producing a photo-electric motive force, such as solar battery. On the rear side of the plate there is formed a light intercepting surface 18.

A return mirror 21 is inclined (for example, by 45 degrees) with the optical axis 7 is arranged on the optical axis 7 to the rear of the focusing mirror 9, in the camera 6 fitted to the rear of the camera adaptor 5. A photographic film 22 is arranged further to the rear on the optical axis.

A pentaprism 23 turning the image laterally back to an erect image is arrange on the reflected optical axis (to the upper side as drawn in the illustration) of the return mirror 21, and a view-finder lens 24 is arranged on the optical axis through this pentaprism 23, so that the optical image as formed on the film 22 can be observed by eye close to the rear of the view finder lens 24. In the case of photography, the return mirror 21 will be retracted so that the incident light can form an image on the surface of the film 22, when an exposure is made.

The position of the photo-electric surface 17 of the transducer element 11 is arranged to be optically conjugate with the position of the surface of the film 22.

That is to say, the light path for light passing through the lens 8 to form an image on the surface of the film 22 and the light path for light reflected by the focusing mirror 9 to form an image on the photo-electric surface 17 of the transducer element 11 through the image reforming lens 10 are mutually equal.

A light guide provided as an illuminating light transmitting means through the insertion sheath 1 of the endoscope 4 is connected with a light-guide supply optical fibre bunch 31 in the light guide mouthpiece 2. Illuminating light from a diagnosis light source 32 is projected onto the input end face of this light-guide 32. In accordance with the present invention the above-mentioned light-guide 31 has a branched portion, so that light is not only transmitted to the mouthpiece 2 of the endoscope 4 but also to the pinhole 15 in the light intercepting surface 18 on the adjacent side of the transducer element 11 to form a light-spot guide 33 to project illuminating light for focus state detection toward an object 25 that is to be recorded, using the pinhole 15 as an aperture for projecting light from the emitting end face of this light-spot guide 33.

The principle of the focus state detecting function in the camera adaptor 5 of this first exemplary embodiment will now be explained with reference to Figure 3, in which the optical system is simplified, to be shown as a linear optical system.

As shown in Figure 3, the light projected onto the input end face of the light-spot guide 33 from the light source 32 will be emitted from the other end to be projected and reflected by the object 25 that is to be recorded, and will return to the photo-sensitive side of the transducer element 11 via the lens 8 and mirror 9. If the object 25 to be recorded is in the position indicated by the reference symbol b, a correctly focused image will be formed, but if the object 25 is too near or too far, then it will be out of focus on return, as indicated by broken lines or chain-dotted lines repectively related to the positions a and c. (The reference symbols a and c correspond respectively to the front focus and rear focus with respect to the objective lens 8).

In the above-mentioned case, the light projected through the pinhole 15 will form a projected light image that is matched to the pinhole-shape, and when the object 25 is in the position b, the light reflected will be convergent to form an image point in the pinhole 15, and therefore no light from the light-spot guide will fall upon the photo-sensitive surface 17 around the pinhole 15. On the other hand, if the object 25 is in the position a the reflected beam is expanded and light reflected will be directed to come to a point to the rear of the pinhole 15, and will therefore strike the photo-electric surface 17 on the outer periphery of the pinhole 15, and produce an output signal. In the like manner, light projected onto an object 25 in the position $c$ will be reflected to form a convergent point in the front of the pinhole 15, and will then expand to reach the photo-electric surface 17, and produce a signal from the photo-electric transducer element.

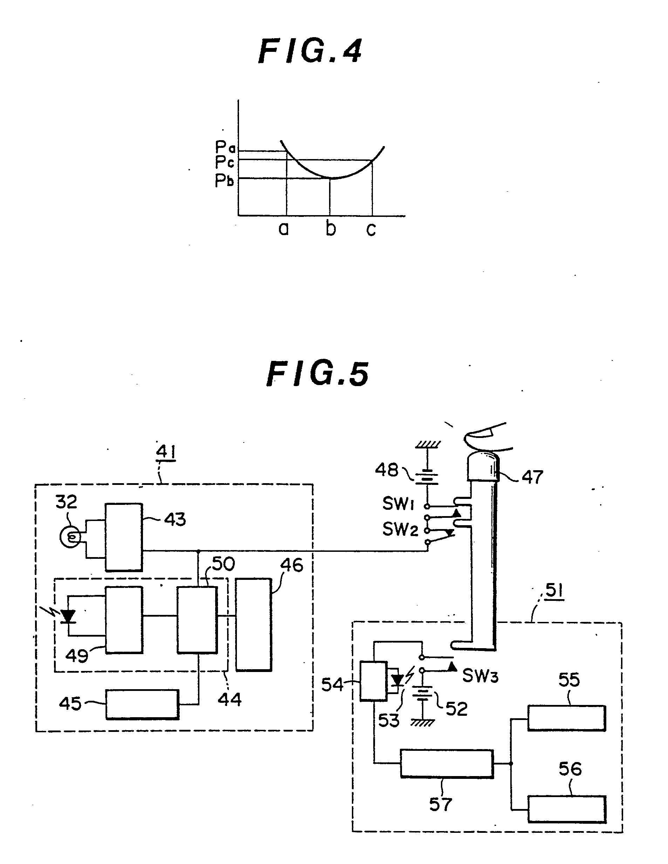

The above description relates to the light from the illuminating diagnosis light source 32 being emitted on paths close to the optical axis 7. In fact, the illuminating light will be projected toward the object 25 over a relatively wide angle, even by the light guide inserted through the endoscope 4. Therefore, the reflected light from a position displaced from the optical axis 7 will be partly incident and the output signal of the photo-electric transducer element 11 may be swamped by the output level of this diagnosis light component as shown in Figure 4 wherein the abscissa represents the positions of the object that is to be photographed and the ordinate represents examples of the detecting output levels of the photo-electric transducer element 11.

Thus, when the object 25 to be photographed is in the correct focus position $b$, the output level Pb of the photo-electric transducer element 11 will be minimum, and if it is nearer or farther than that, the respective signal output levels Pa and Pc will be larger.

Thus, the position in which the signal output is minimum will indicate the correct focus position. Therefore, if a camera is used with the objective lens 8 in the position in which the output of the photo-electric transducer element 11 is at a minimum, as determined by moving the lens 8 with respect to the object 25 at any distance, a clearly focused image (photograph) can be recorded. If the lens 8 is moved, the direction in which the output level becomes smaller will be recognised as that which approaches the correct focusing state, and therefore the lens 8 can be manually or automatically moved in this direction. In the case of automatic setting of the focus, if the movement is stopped at a point where the output level begins to increase, and centred at the point where there is substantially no change with movement of the lens 8, the photographic lens 8 can be set in the correct focus position for an object 25 at any distance.

The above described principle can be applied in exactly the same way to an optical system within the camera 6 shown in Figure 1, except that the image reforming lens 10 and focusing mirror 9 must then be arranged on a path to the photo-electric transducer element 11 to the rear of the camera objective lens and the endoscope 4 is arranged on the path to the object 25 in front of the camera objective lens.

In the camera adaptor 5 shown in Figure 1, when correct focusing is achieved, a focus state indicating lamp 14 will light and light from this lamp will be condensed by a lamp lens 13, reflected by the return mirror 9, to proceed rearwardly on the optical axis 7 and be incident upon the eye of an observer through the return mirror 21, pentaprism 23 and view-finder 24. Therefore, if an exposure is made when the focus state indicating lamp 14 lights, a clear image will be recorded.

The illuminating light for the light-spot, in accordance with the invention, if fed through the light-spot guide 33 from the external illuminating diagnosis light source 32. Therefore, an illuminating beam of a sufficient intensity can be projected toward the object 25 to be recorded without being limited by the space available within the camers adaptor 5 or the like. Therefore, the detecting output level of the photo-electric transducer element 11 will be better than an arrangement requiring the light-source to be within the adaptor, and the detecting sensitivity and signal-to-noise ratio (SN ratio) will also be improved relative to the latter arrangement.

Figure 5 shows a block schematic diagram of the circuit arrangement for an endoscope focus setting device 41 whereby the lens 8 is automatically moved and set at the correct focus point by using the first described focus state detector, together with an automatic exposure device.

The focus setting device 41 comprises a light source power supply 43 which energises the light source 32 to emit light to be projected onto the object 25 that is to be photographed or otherwise recorded. A focus state detector circuit 44 amplifies any signal output produced by light that is reflected from the object 25 by the photo-electric transducer element 11 provided with an aperture such as the pinhole 15, and detects whether focus is correct or not. An indicator 45 (corresponding to the above mentioned focus indicating lamp 14) indicates the instant when focusing is correct, after an objective lens driving device 46 has caused the lens 8 to move to the required focus position.

The above mentioned light emitting circuit 43 is so formed that, for example, when a release button 47 is pushed, a normally open switch SW1 will be closed, whilst a normally closed switch SW2 remains on to feed electrical power from a current source 48 (indicated by a battery) to energise the light source 32, and illuminating light for focus state detection will be emitted through the pinhole 15 from the back of the photo-electric transducer element 11 via the light-spot guide 33 from this light source 32.

The focus state detector circuit 44 comprises an amplifier 49 amplifying the output signal of the photo-electric transducer element 11, and a comparator detecting circuit 50. This comparator detecting circuit 50 is so formed that if the driving device 46 moves the lens 8 forward or rearward at a fixed velocity, for example, detectig signals (amplified by the amplifier 49) will be fed in in sequence at given intervals, and in each case the previous signal value compared with the signal value now applied, until the later signal is equal to or larger than the previous signal, when a sensing signal is produced to stop the driving device 46 and move the lens 8 in the reverse direction, until the sensing signal is set constant for a given time, the signal being integrated and the integrated output is above a fixed value, or when the number of sensing signals fed in within a fixed time is counted and the counted number found to be above a given value, the indicator 45 will be lighted and the operation of the driving device 46 will be stopped.

On the other hand, when the release button 47 is pushed further down, a third, normally open switch SW3 will be closed and the second switch SW2 opened. so that the endoscope focus setting device 41 will not operate but the third switch SW3 will operate the automatic exposing (EE) device 51 now to be described.

This EE device 51 comprises an amplifier 54 wherein, when the switch SW3 is closed and, electric power fed from a current source 52, and any detecting signal from a photo-sensitive photo-electric transducer element 53 will be amplified and fed to an EE control circuit 57 which controls respectively the shutter speed of an EE shutter 55 and a light intensity control circuit 56 controlling the light output of the light source 32, so that when the switch SW3 is closed the EE control circuit 57 will operate to adjust the shutter speed and light output.

In the focus setting device 41 formed as described above, the lens 8 is moved and set in the correct focus position by an operation now to be described.

When the release button 47 is pushed to close the switch SW1, the light source 32 will be energised and this light will be transmitted through the light-spot guide 33, pass through the pinhole 15, be reflected by the focusing mirror 9, passed through the lens 8 and endoscope 4 and projected as a spot onto the object 25.

The light reflected by the object 25 will pass back through the lens 8, to be reflected by the focusing mirror 9 and proceed to the photo-electric transducer element 11 provided with the pinhole 15. If the above mentioned lens 8 is in the correct focus position for the object 25 to be clearly photographed, or recorded the output level of the photo-electric transducer element 11 will be at a minimum value. Therefore, in this case, when the driving device 46 moves the lens 8, forward for example, the output level will become larger, and therefore the advancing operation will be immediately stopped, a sensing signal will be put out to the comparator detecting circuit 50, and the driving device 46 will move the lens 8 in the reverse direction. Again, the output level will become larger, and therefore the sensing signal will be emitted, the operation of moving the lens 8 rearward will be stopped, the lens 8 will be moved in the reverse direction (that is, forward) and will thus slightly vibrate on the optical axis 7. In this case, the sensing signal will be repeatedly emitted by the comparator detecting circuit 50, and therefore, if it is fed to an integrating circuit having a proper time constant, or to a counting circuit, the output will rise above a given value and the vibrating action will be stopped.

On the other hand, if the objective lens 8 is away from the correct focusing positiion initially, if the driving device 46 moves the photographic lens 8 in the direction taking it further from the focusing position, the output level of the photo-element 11 will gradually increase and therefore the comparator detecting circuit 50 will control the driving device 46 to move the lens 8 in the reverse direction, as is described above. When the lens 8 passes through the correct focus position, the slight vibrating operation as described above, will be initiated and the lens 8 will finally be set in the correct focus position.

When the lens 8 is thus correctly set the indicator 45 will cause the focusing indicating lamp 14 to light. When the observer sees this, by further pushing the release button 47, the EE device 51 can be caused to operate and a photograph or recording will be made. The EE device 51 is operated simultaneously in this embodiment, when the button 47 operates the switch contact SW3, but in some cases it may be advantageous to have the EE device continuously operating. The indicator 45 may be made to operate a buzzer or like transducer instead of lighting the lamp 14.

In this first embodiment, as part of the light guide 31 transmitting the illuminating light from the light source 32 is utilised for the light-spot focusing light, an adequately intense focusing illumination can be projected onto the object 25 without increasing the volume and weight of the adaptor device, whilst the output level of the photo-electric transducer element 11 and the SN ratio will be better than that obtained if a separate light source of limited size is provided within the adaptor, and the focus state can be detected without impairing the operating function even in an endoscope of a dark optical system. The light-spot guide 33 of the light projecting means extends out of the adaptor, but the focus detecting part with the photo-electric transducer element 11 in which the pinhole 15 is formed can readily be contained within a small space. As the first embodiment has such a simple construction it can be realised at a low cost.

As the light projecting means is provided, if the intensity of the light to be projected is adjusted, even in case a dark optical system is to be used, focusing will be able to be detected at a high precision.

The focus setting device using the first embodiment sets the lens 8 so that the output level of the light receiving photo-electric transducer element 11 may be of a minimum value and therefore, the lens 8 can be set in a correctly focused state with high precision to obtain a clear recorded image without impairing the operating function.

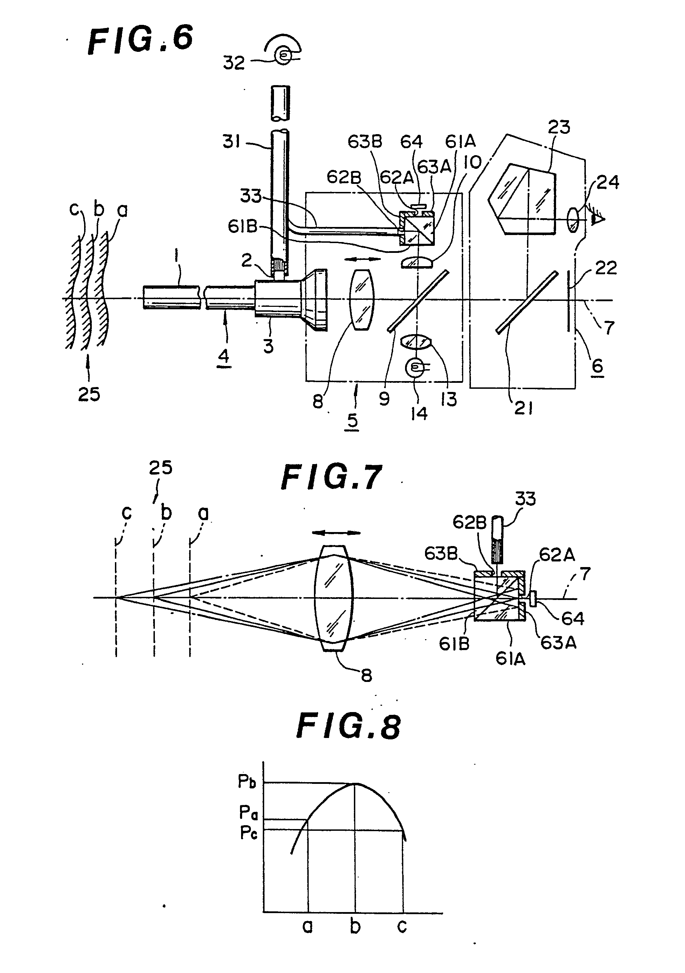

Figure 6 shows the optical system of a second exemplary embodiment of the present invention, in which modified means are provided for projecting and receiving the focus state detecting light.

A beam splitter, formed by jointing two right prisms 61A and 61B for example, is arranged to the rear (upper side in the illustration) of an image reforming lens 10. Light intercepting plates 63A and 63B with respective pinholes 62A and 62B are bonded on adjacent surfaces of the right prism 61A and 61B so that the respective pinholes are to the rear of the optical axes passing through and reflected by the jointing surface of these right prisms 61A and 61B, a photo-electric transducer element 64 is positioned on the axis after the pinhole 62A, and the emitting end face of the light spot guide 33 is arranged to the rear of the pinhole 62B.

The pinhole 62A serves as a light receiving aperture in front of the photo-electric transducer element 64, and is set in a position optically conjugate with the film surface 22.

The principle of operation for focus state detection used in this second embodiment is illustrated in Figure 7.

The focus state detecting light transmitted by the light-spot guide 33 from the light source 32 passes through the pinhole 62B provided in one light intercepting plate 63B, to be reflected by the joining surface of the prisms 61A and 61B and then projected toward the object 25 from the mirror 9 through lens 8. The light reflected from the object 25 will pass back through the lens 8 and the prisms 61B and 61A, to be incident upon the other light intercepting plate 63A. If the lens 8 is in a correct focusing position, a convergent point will be formed at the position of the pinhole 62A, and therefore almost all of the reflected light will pass through the pinhole 62A to reach the photo-electric transducer element 64. If the lens 8 is not in a correct focusing position, the light passing through the pinhole 62A to reach the photo-electric transducer element 64 will be reduced. The output characteristics of the photo-electric transducer element 64 will be as shown in Figure 8, as the output level for the correct focusing position will be a maximum value. The construction of the second embodiment is otherwise as described for the first embodiment, and the same reference numerals are used respectively for those elements that are identical in these two embodiments, and for those in the other embodiments yet to be described.

This second embodiment has substantially the same general operation and effect as the first embodiment. However, as compared with the above described embodiment, as separate light receiving and projecting apertures are provided, in the light intercepting plates 62A and 62B respectively, there is an advantage that no aperture needs to be provided in the photo-electric transducer element 11, which is comparatively hard to work.

The beam splitter in the second embodiment can be formed by a semi-transparent mirror instead of the prisms 61A and 61B. An endoscope automatic focusing device can be formed in the manner described for the first embodiment, but with the circuitry designed to seek the maximum output from the transducer.

The respective pinholes, 62A and 62B, provided in the light intercepting plates 63A and 63B, may be apertures of any shape. The apertures in the first embodiment and this embodiment need to be optically transparent, but may be physically closed, i.e. glass or like material through which the light will pass may be present.

If the filter is provided on the end face of the light spot guide 33 in the first and second embodiments, to selectively pass light of a specific wave length, the photo-electric transducer elements 11 or 64 are of high sensitivity to light of this wave length the SN ratio will be improved.

In the first and second embodiments, the endoscope focus state detecting focus setting device is formed within the camera adaptor 5 for a photographic or television camera to be fitted to the endoscope 4. However, the device may be contained within a camera designed (for endscopes) and not requiring an adaptor.

## Claims

1. An endoscope having a focus state detector (11), means being provided to transmit illuminating light from a light source (32) to a light-beam trasmitting means within an endoscope via a light guide and a light-spot guide to project a light spot on to an object that is to be recorded as an optical image, the image of the illuminated object being passed back to a predetermined image forming surface via an observing optical system in the endoscope and thence through an adjustable lens system whose focusing state is monitored by said detectopr, which comprises a light receiving means using a photoelectric transducer element to assess whether there is a clearly focused image at said image forming surface, the light returning from said projected light-spot being directed to an optical aperture or locally non-sensitive part of said transducer having a shape and size matching that of the reflected light-spot formed in a position optically conjugate with said image forming surface, the light-emitting end face of said light-spot guide either facing an optical aperture (15) formed in the light receiving means or an optical aperture (62A) adjacent to the light receiving means, characterised in that the light-spot guide is formed by a branching (33) of the light guide that projects its light via a lens (10) common to the path of the returning light.

2. An endoscope as claimed in Claim 1, characterised in that said light-spot guide (33) formed by part of the light guide (31) transmits light selectively to be projected on the spot via a pinhole (15;62B) arranged in the path of the light from the illuminating diagnosis light source (32) emitted by said light-spot guide (33).

3. An endoscope as claimed in Claim 1 or Claim 2, characterised in that said light receiving means (17;64) is contained in a camera adaptor (5) removably fitted to the endoscope (4).

4. An endoscope as claimed in any preceding Claim, characterised in that said light-spot is transmitted to be reflected back through a light guide and form an image of said spot on said transducer element through a movable lens system (8), with a driving means (46) automatically moving and setting said lens system under the control of the output of said transducer element so that a clear image is formed.

5. An endoscope as claimed in Claim 5, characterised in that said driving means (46) is controlled to move the lens system (8) until the output level of said transducer has a unique value, which may be maximum or minimum.

6. An endoscope as claimed in Claim 6, characterised in that the instant at which the output level of said transducer is at said unique value is confirmed by an indicating means (45).

**Patentansprüche**

1. Endoskop mit einem Fokuszustandsdetektor (11), bei dem Mittel vorgesehen sind zur Übertragung von Beleuchtungslicht von einer Lichtquelle (32) zu Lichtbündelübertragungsmitteln in einem Endoskop über eine Lichtführung und eine Lichtpunktführung, um einen Lichtpunkt auf ein Objekt zu projizieren, das als optisches Bild aufgenommen werden soll, wobei das Bild des beleuchteten Gegenstandes zurück auf eine vorbestimmte Abbildungsfläche über ein optisches Beobachtungssystem in dem Endoskop und dabei durch ein einstellbares Linsensystem geführt wird, dessen Fokuszustand durch den Detektor angezeigt wird, wobei dieser einen Lichtempfänger mit einem fotoelektrischen Wandlerelement aufweist zum Abschätzen, ob ein klar fokussiertes Bild auf der Abbildungsfläche vorliegt, wobei ferner das von dem projizierten Lichtpunkt zurückkommende Licht auf eine optische Öffnung oder einen lokalen nicht empfindlichen Teil des Wandlers gerichtet wird, die (der) eine solche Form und Abmessung aufweist, daß der reflektierte Lichtpunkt abgeglichen wird, der in einer optischen konjugierten Position mit der Abbildungsfläche gebildet wird und wobei die lichtemittierrende Stirnfläche der Lichtpunktführung entweder einer optischen Öffnung (15) in dem Lichtempfänger oder einer optischen Öffnung (62a) nahe dem Lichtempfänger zugekehrt ist, dadurch gekennzeichnet, daß die Lichtpunktführung durch eine Abzweigung (33) der Lichtführung gebildet ist, die ihr Licht über die Linse (10) gemeinsam mit dem Weg des reflektierenden Lichtes projiziert.

2. Endoskop nach Anspruch 1, dadurch gekennzeichnet, daß die Lichtpunktführung (33), die durch einen Teil der Lichtführung (31) gebildet ist, das Licht, das selektiv auf einen Punkt projiziert wird, über ein Loch (15, 62b) in den Weg des Lichtes von der Beleuchtungslichtquelle (32) emittiert von der Lichtpunktführung (33) übertragt.

3. Endoskop nach Anspruch 1 oder 2, dadurch gekennnzeichnet, daß die Lichtempfänger (17, 64) in dem Kameraadapter (5) enthalten sind, der entfernbar mit dem Endoskop verbunden ist.

4. Endoskop nach einem der vorstehenden Ansprüche, dadurch gekennzeichnet, daß der Lichtpunkt durch eine Lichtführung reflektiert wird und ein Bild des Punktes aug dem Wandlerelement durch das bewegliche Linsensystem (8) bildet, wobei der Antrieb (46) automatisch die Linse unter der Kontrolle des Ausganges dieses Wandlerelementes bewegt und einstellt, so daß ein klares Bild gebildet wird.

5. Endoskop nach Anspruch 4, dadurch gekennzeichnet, daß der Antrieb (46) so gesteuert wird, daß er das Linsensystem (8) solange bewegt, bis der Ausgangswert des Wandlers einen einheitlichen Wert, der ein Maximum oder Minimum sein kann, aufweist.

6. Endoskop nach Anspruch 5, dadurch gekennzeichnet, daß der Zustand, in dem sich der Ausgangswert des Wandlers bei diesem einheitlichen Wert befindet, durch eine Anzeige (45) bestätigt wird.

**Revendications**

1. Endoscope ayant un détecteur de mise au point (11), des moyens étant prévus pour transmettre la lumière d'éclairage d'une source lumineuse (32) jusqu'à des moyens transmetteurs de faisceaux lumineux à l'intérieur d'un endscope par un guide lumineux et un guide de points lumineux pour projeter un point lumineux sur un objet qui doit être enregistré sous forme d'images optiques, l'image de l'objet éclairé étant remenée sur une surface prédéterminée de formation d'images par un système optique d'observation faisant partie de l'endoscope et pour cela à travers un système à lentille réglable dont l'état de mise au point est surveillé par ledit détecteur, lequel comprend des moyens récepteurs de lumière utilisant un élément capteur photo-électrique pour juger s'il y a une image bien mise au point sur ladite surface de formation d'images, la lumière réfléchie par ledit point lumineux projeté étant dirigée vers une ouverture optique ou une partie localement insensible dudit capteur ayant une forme et des dimensions qui correspondent audit point lumineux réfléchi formé dans une position qui est le conjugué optique de ladite surface de formation d'images, la face de l'extrémité émetteur de lumière dudit guide de points lumineux étant tournée soit vers une ouverture optique (15) formée dans les moyens récepteurs de lumière, soit vers une ouverture optique (62a) adjacent aux moyens récepteurs de lumière, caractérisé par le fait que le guide de points lumineux est formé par une dérivation (33) de guide lumineux qui envoie sa lumière par une lentille (10) commune au trajet de retour de la lumière.

2. Endoscope selon la revendication 1, caractérisé par le fait que ledit guide de points lumineux (33) formé per une partie du guide lumineux (31) transmet la lumière de façon sélective pour la projeter sur le point par un minuscule orifice (15; 62b) prévu sur le trajet de la lumière provenant de la source lumineuse de diagnostic d'éclairage (32) émise par ledit guide de points lumineux (33).

3. Endoscope selon la revendication 1 ou la revendication 2, caractérise par le fait que lesdits moyens récepteurs de lumière (17; 64) sont logés dans un adaptateur d'appareil de prise de vues (5) installé de façon amovible sur l'endoscope (4).

4. Endoscope selon l'une quelconque des revendications précédentes, caractérise par le fait que ledit point lumineux est transmis pour être réfléchi par un guide lumineux et former une image dudit point sur ledit élément capteur à travers un système à lentille amovible (8), avec un moyen d'entraînement (46) déplaçant et réglant automatiquement ledit système à lentille sous le contrôle de la sortie dudit élément capteur afin qu'une image nette soit formée.

5. Endoscope selon la revendication 5, caractérisé par le fait que lesdits moyens d'entraînement (46) sont commandés pour déplacer le système à lentille (18) jusqu'à ce que le niveau de sortie dudit capteur ait une valeur unique, qui peut être maximale ou minimale.

6. Endoscope selon la revendication 6, caractérisé par le fait que l'instant auquel le niveau de sortie dudit capteur atteint ladite valeur unique est confirmé par un moyen indicateur (45).

# FIG.1

# FIG.2

# FIG.3

1

# *FIG.4*

# *FIG.5*

# FIG.6

# FIG.7

# FIG.8